# EUROPEAN PATENT SPECIFICATION

(11) **EP 0 534 088 B1**
(45) Date of publication and mention of the grant of the patent: **29.11.1995**
(21) Application number: 92112826.0
(22) Date of filing: 28.07.1992
(51) Int. Cl.: B05B 11/00, B05B 1/34, B65D 83/14, A61M 15/08

(54) **Nasal dispenser of atomized pharmaceutical substances**
Nasenspray
Pulvérisateur nasal

(30) Priority: 24.09.1991 IT MI912546
(43) Date of publication of application: 31.03.1993
(73) Proprietor: ELETTRO PLASTICA S.p.A., I-20089 Rozzano, Milan (IT)
(72) Inventor: Marelli, Andrea, I-20089 Rozzano, Milan (IT)
(74) Representative: Giambrocono, Alfonso, Dr. Ing.

(56) References cited:
- EP-A- 0 131 501
- US-A- 4 220 285

## Description

The present invention relates to a nasal dispenser for atomized pharmaceutical substances.

Nasal dispensers for atomized pharmaceutical substances are known from a long time.

They are mounted on a container of the substance to be administered and comprise, essentially, a valve or a little supplying pump connected to the container mouthpiece and a hollow stem apt to the substance discharge.

Said stem, having to be introduced through the nostrils into the nasal cavities has a thin and lengthened shape and it is gone through by a supplying channel.

It is necessary that the above mentioned channel, as it is known to the skilled in the art, is as little as possible, in such a way that one obtains minimum stagnations of the substance to be administered.

The carrying out, in great quantity and at a very low cost, through automatic machines, of dispensers having a long channel with a very little transversal section has however revealed itself practically impossible.

To solve such a problem we have thought (see for example EP-A-0131501 and US-A-4801093) to produce dispensers having a discharge channel with a transversal section showing such a size to allow an automatic, fast and precise production, and to lately introduce in said channel, a peg which limits the wideness thereof, and allows the discharge of the pharmaceutical substance alone from one or more extremely thin channels having a limited section, obtained conforming opportunely the peg with respect to the channel surface.

For instance, in the already cited EP-A-0131501, a constant section peg is forcedly introduced, into the discharge channel, which has a uniform circular transversal section, said peg being as long as said channel and with its external surface showing, for all its longitudinal extension, a flat part which defines a little section passage with the opposite surface of the channel apt to a perfect supplying of the pharmaceutical substance without stagnations.

Also such a solution has very serious drawbacks.

In fact, the peg has to be forcedly inserted in the discharge channel, in such a way that, after it is placed, it results stablely and univocally kept in the above mentioned channel and it cannot move or be moved in any way, such a thing causing a sure malfunction of the dispenser.

The here above said insertion is a difficult and delicate operation, which requires a high precision manufacture, which definitely slows the production times and makes the production costs increase.

The above said peg is, in fact, a substantially long and thin member, at least a part of whose external surface, when the peg is inserted in the channel, flowing with a remarkable friction, all along the peg, on the channel surface.

Therefore, during the insertion the peg is extremely stressed, and hence to avoid its breaking or its damaging, as already said, it is required that the dispenser manufacturing procedure has a high precision, accordingly slow and high priced.

The purpose of the present invention is that one to realize a Peg for dispensers of the above mentioned type which is easy, fast and sure to be inserted in the discharge channel of the dispensers, thus dramatically lowering costs and production times of the above said dispensers.

These and other purposes, which will result evident to the skilled in the art, are reached by a dispenser according to claim 1.

Said appendixes are conveniently some little teeth placed along one or more perimetric lines lying in a plane that is perpendicular to the longitudinal axis of the element placed in the dispenser channel.

For a better comprehension of the present invention the following drawing has been enclosed, by non limiting way of example, wherein:
- Figure 1 represents the dispenser according to its longitudinal section;
- Figure 2 is a perspective view, in an enlarged scale with respect to that one of Figure 1, of the shaped element inserted in the dispenser discharge channel;
- Figure 3 is a section, in an enlarged scale, with respect to that one of Figure 1, taken according to the line 3-3 of Figure 1.

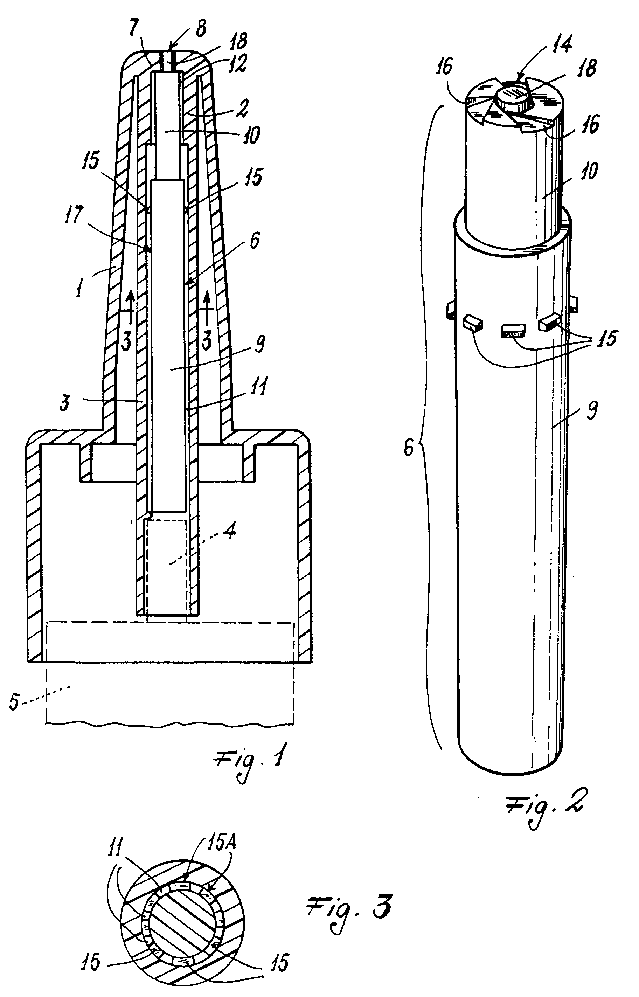

The nasal dispenser illustrated in Figure 1 comprises a lengthened external wall 1, slightly conic, and an internal tubular wall made up by two wall 2 and 3 parts, consecutive between themselves, having circular transversal sections but of a different diameter and defining a unique lengthened channel 17.

Said elements are made of plastics.

The free end of the wall 3 part can be pierced on its end, by a stem 4 being part of a little pump having manual functioning or the like, applied on a container 5 wherein the atomized substance meant to be dispensed through the dispenser and to be therefore inhaled by the user, is kept.

A head wall 7 (perpendicular to the inhalator axis) is provided at the top (with respect to Figure 1) of the tubular wall 2, a circular hole 8 being made in said wall.

In the inside of the cavity of the tubular wall 2, 3 it has been inserted and stablely housed a shaped element 6, having circular transversal sections, made by two consecutive parts 9, 10 having different diameters; however lower rispectively than that one of the cross sections of the cavity 17, delimited by the tubular walls 3 and 2, and compenetrated by said parts 9, 10.

Eight appendixes or little teeth 15 (sized in such a way to stablely keep the shaped element after that the said element has been inserted in the inside of the cavity delimited by the portions 3 and 2 are on the external surface of the part 9 of the shaped element 6.

In fact, as it is illustrated in Figure 3, the little teeth 15 adhere perfectly with their external surface 15A (Figure 3) to the opposite surface of the cavity 17, delimited by the part 3 of the tubular wall.

The little teeth 15 have rectangular trapezoidal cross section with the inclined side put in such a way to help the insertion of the element 6 in the above said channel and to oppose itself to the extraction of said element from said channel.

Said inclination reduces in fact the friction of little teeth themselves on the internal wall of the cavity when the shaped element 6 is therein inserted.

The appendixes 15, further to keeping the element 6 in its correct position, keep it spaced from the internal wall of the cavity delimited by the part 3, thus originating a passage 11 through which the substance to be dispensed can flow .

The cylindrical part 10 of the shaped element 6 has a cross section that is smaller than that one of the cavity delimited by the tubular wall 2, so that an annular cylindrical space 12 (Figure 1) is formed between them, which allows the free downflow of the substance to be dispensed.

It can be noticed from Figure 2 that on the top end (with respect to the figures 1 and 2) of the shaped element 6 a recess 14 is obtained, from whose centre a peg 18 projects, extending itself to the centre of the hole 8 made in the end wall 7, leaving an annular space for the discharging of the atomized substance.

The peg 18 penetrating into the hole 8, permits a self centring of the shaped element 6, thus cooperating with the little teeth 15 for the correct housing and keeping of the above said element in the cavity 17 delimited by the walls 2 and 3.

The recess 14 communicates with the annular cylindrical space 12 through sunken parts 16, having a substantially triangular section, showing a side that is substantially tangent to the edge of the recess itself, which permits to give to the little drops of the liquid (coming from the container 5 through the stem 4, the passages 11, the annular passage 12 and the channels 16) a whirling motion in the annular chamber around the peg 18.

The little drops come out therefore under finely and uniformely atomized form, turning round the peg 18, through the space existing between the peg 18 surface and the adjacent surface of the hole 8 made in the end wall 7.

A variation (not illustrated) of the present form of the carrying out provides a shaped element 6 having, in proximity of the lower end (with respect to the figures 1 and 2), a second line of little teeth entirely similar to the teeth 15 before described and illustrated in the figures.

Said second line of little teeth and eventual further lines, are placed along perimetral lines of the shaped element 6 lying in planes perpendicular to the longitudinal axis of the element itself.

Said further lines of little teeth, improve the coupling of the shaped element 6 with the internal wall of the dispenser.

Further they permit an optimal centring of the above said shaped element 6 into the cavity also for those shaped elements which do not have at their ends a peg 18 cooperating with a hole of the dispenser body centred with respect to the cavity.

Furthermore it is understood that the particular shape of the components of the dispenser, wich have been up to now described and illustrated in the figures, has been given merely by way of example.

Thus, for instance, the appendixes or the little teeth 15 of the shaped element 6 might have a different form, e.g., they could have both side walls inclinated in such a way to help the insertion and the keeping of the element in the cavities, or they might be more longitudinally lengthened, or they might be some projecting lines with respect to the external surface of the element 6 and having a curvilinear development.

Even the shape of the element 6 and of the cavity 17 delimited by the walls 3 and 2 might be different and not necessarily regular and/or complementary among themselves.

Besides , the end 14 of the element 6 might be flat bearing the device giving the little drops a whirling motion, comprised in the body 1 of the dispenser, in the proximity of the hole for the discharging of the pharmaceutical substance.

The insertion in the channel delimited by the walls 3 and 2 of the shaped element 6 having the appendixes 15 results extremely simplified with respect to the previous assembling operations of the kown dispensers.

The surface of the shaped element which has to graze against the walls of the discharging channel 17 is extremely reduced utilizing a shaped element having some appendixes, reducing, as a consequence, the stresses derived by the friction between the two above said surfaces and therefore making the dispenser assembling faster, simpler and easier.

## Claims

1. Nasal dispenser of atomized pharmaceutical substances, comprising a discharge channel (17) of the pharmaceutical substance, in said discharge channel (17) being housed a lengthened shaped element (6) made of plastics, said shaped element (6) having appendices (15) projecting from its external surface, **characterized** in that said appendices (15) adhere perfectly with their external surfaces (15A) to the internal surface of the tubular discharge channel (17) for retaining the shaped element (6) in a stable position therein.

2. Nasal dispenser according to claim 1, characterized in that said appendices (15) are placed along a common perimetric line of the shaped element (6), said line lying in a plane perpendicular to the longitudinal plane of the shaped element.

3. Nasal dispenser according to claim 1, characterized in that said appendices (15) are placed along at least two perimetric lines of the shaped element (6), said lines lying in planes perpendicular to the longitudinal axis of the shared element.

4. Nasal dispenser according to claims 1 to 3, characterized in that said appendices (15) are little teeth being spaced from each other along the side external surface of the shaped element (6).

5. Dispenser according to claim 4, characterized in that said little teeth (15) are inclinated in such a way as to help the insertion of the shaped element (6) in said channel (17) and to oppose to the extraction of said element (6) from said channel (17).

## Patentansprüche

1. Nasalspender für zerstäubte pharmazeutische Substanzen, umfassend einen Entladungskanal (17) für die pharmazeutische Substanz, wobei in dem Entladungskanal (17) ein aus Kunststoff hergestelltes gestrecktes, geformtes Element (6) untergebracht ist, wobei das geformte Element (6) Ansätze (15) hat, die von seiner äußeren Oberfläche vorstehen, dadurch **gekennzeichnet,** daß die Ansätze (15) perfekt mit ihren äußeren Oberflächen (15A) an der inneren Oberfläche des rohrförmigen Entladungskanals (17) zum Halten des geformten Elements (6) in einer stabilen Position darin haften.

2. Nasalspender gemäß Anspruch 1, dadurch **gekennzeichnet,** daß die Ansätze (15) längs einer gemeinsamen Umfangslinie des geformten Elements (6) plaziert sind, wobei die Linie in einer Ebene liegt, welche senkrecht zu der Längsebene des geformten Elements ist.

3. Nasalspender gemäß Anspruch 1, dadurch **gekennzeichnet,** daß die Ansätze (15) längs wenigstens zweier Umfangslinien des geformten Elements (6) plaziert sind, wobei die Linien in Ebenen liegen, die senkrecht zu der Längsachse des geformten Elements sind.

4. Nasalspender gemäß den Ansprüche 1 bis 3, dadurch **gekennzeichnet,** daß die Ansätze (15) kleine Zähne sind, die voneinander längs der seitlichen äußeren Oberfläche des geformten Elements (6) beabstandet sind.

5. Spender gemäß Anspruch 4, dadurch **gekennzeichnet,** daß die kleinen Zähne (15) in einer solchen Art und Weise geneigt sind, daß sie das Einfügen des geformten Elements (6) in den Kanal (17) unterstützen und dem Herausziehen des Elements (6) aus dem Kanal (17) entgegenwirken.

## Revendications

1. Pulvérisateur nasal de substances pharmaceutiques atomisées, comportant un canal de distribution (17) de la substance pharmaceutique, un élément profilé allongé (6) en matière plastique étant logé dans ledit canal de distribution (17), ledit élément profilé (6) portant des aspérités (15) qui font saillie de sa surface extérieure, caractérisé en ce lesdites aspérités (15) adhèrent parfaitement par leurs surfaces extérieures (15A) à la surface intérieure du canal tubulaire de distribution (17) de manière à maintenir l'élément profilé (6) en position stable à l'intérieur.

2. Pulvérisateur nasal selon la revendication 1, caractérisé en ce que lesdites aspérités (15) sont disposées le long d'une ligne périphérique commune de l'élément profilé (6), ladite ligne étant située dans un plan perpendiculaire au plan longitudinal de l'élément profilé.

3. Pulvérisateur nasal selon la revendication 1, caractérisé en ce que lesdites aspérités (15) sont placées le long d'au moins deux lignes périphériques de l'élément profilé (6), lesdites lignes étant situées dans des plans perpendiculaires à l'axe longitudinal de l'élément profilé.

4. Pulvérisateur nasal selon les revendications 1 à 3, caractérisé en ce que lesdites aspérités (15) sont des petites dents espacées l'une de l'autre le long de la surface latérale extérieure de l'élément profilé (6).

5. Pulvérisateur selon la revendication 4, caractérisé en ce que lesdites petites dents (15) sont inclinées de manière à faciliter l'introduction de l'élément profilé (6) dans ledit canal (17), et à s'opposer à l'extraction dudit élément (6) pour le sortir dudit canal (17).
